# NEUE EUROPÄISCHE PATENTSCHRIFT

(11) **EP 0 659 767 B2**
(45) Veröffentlichungstag und Bekanntmachung des Hinweises auf die Entscheidung über den Einspruch: **06.11.2002**
(45) Hinweis auf die Patenterteilung: 20.01.1999
(21) Anmeldenummer: 93810912.1
(22) Anmeldetag: 27.12.1993
(51) Int. Cl.: C07K 16/34, C07K 16/06, A61K 39/395

(54) **Verfahren zur Herstellung eines Konzentrates von Anti-D-Immunoglobulin G und pharmazeutische Zusammensetzung, die dieses enthält**
Process to prepare an anti-D immunoglobulin G concentrate and pharmaceutical compositions comprising it
Procédé de préparation d'un concentré d'immunoglobuline G anti-D et compositions pharmaceutiques qui le contiennent

(43) Veröffentlichungstag der Anmeldung: 28.06.1995
(73) Patentinhaber: ZLB Bioplasma AG, 3014 Bern (CH)
(72) Erfinder: Stucki, Martin Dr.,, CH-3177 Laupen, (CH); Lerch, Peter Dr.,, CH-3007 Bern, (CH); Hodler, Gerhard,, CH-3076 Worb, (CH)
(74) Vertreter: Fischer, Franz Joseph

(56) Entgegenhaltungen:
- EP-A- 0 085 747
- EP-A- 0 440 483
- WO-A-89/05157
- US-A- 3 869 436
- JOURNAL OF APPLIED BIOCHEMISTRY Bd. 3 , 1981 , NEW YORK Seiten 164 - 175 FRIESEN A.D. ET AL 'Column ion-Exchange preparation and characterisation of an Rh immune globulin (WinRho) for intravenous use'
- THROMBOSIS AND HAEMOSTASIS Bd. 65, Nr. 6 , 1991 , NEW YORK Seite 1163 HOROWITZ, S. ET AL 'Preparation and characterisation of S/D-FFP, a virus sterilised "Fresh frozen plasma"'
- JAMES E.F. REYNOLDS 'Martindale, The extra Pharmacopoeia' 1989 , THE PHARMACEUTICAL PRESS , LONDON * Seite 811 - Seite 812 * * Seite 1170 - Seite 1171 *
- BUNDESVERBAND DER PHARMACEUTISCHEN INDUSTRIE E.V. 'Rote Liste' 1992 , EDITIO CANTOR , AULENDORF/WÜRTT. *Zusammensetzungen 74030, 74031, 74033, 74034*
- Vox Sang, Vol 24, 1973, Seiten 301-316

## Beschreibung

Die vorliegende Erfindung betrifft ein Verfahren zur Herstellung eines neuen Konzentrates von Anti-D (Anti-Rhesus) Immunglobulin G (Anti-D IgG), sowie eine pharmazeutische Zusammensetzung, die ein solches Konzentrat als aktives Prinzip enthält.

Als Morbus haemolyticus neonatorum bezeichnet man allgemein die hämolytische Anämie bei Foeten und Neugeborenen, die durch mütterliche Antikörper hervorgerufen wird. Diese sind gegen Antigene auf der Oberfläche der kindlichen Erythrozyten gerichtet. Es handelt sich dabei entweder um Antigene des Rhesus-, des ABO- oder anderer Blutgruppensysteme.

Die wichtigsten Rhesus-Blutgruppenantigene sind D, die deutlich weniger immunogen wirksamen antithetischen Antigene C,c und E,e sowie D^{u}; daneben sind über 40 weitere Rhesus-Antigene bekannt. Von klinischer Bedeutung bei der Rhesus-Unverträglichkeit ist vor allem das Rhesus-Antigen D (RhD; Rhₒ), ein Membranprotein der Erythrozyten, welches kürzlich kloniert und dessen Primärstruktur beschrieben wurde (Le Van Kim et al. PNAS USA, 89,10925,1992). Das D-Antigen kommt bei ca. 85% der Weissen in Europa vor; diese Individuen werden als Rhesus-positiv bezeichnet. Individuen, denen das Antigen D fehlt werden als Rhesus-negativ bezeichnet. Antikörper der Spezifität Anti-D sind die häufigsten irregulären Rhesus-Antikörper und treten vor allem während Rhesus-inkompatibler Schwangerschaften und nach Transfusion Rhesus-inkompatiblen Blutes auf.

Eine sichere wirksame kausale Therapie bei Rhesus-sensibilisierten Schwangeren ist bisher nicht bekannt. Da eine Desensibilisierung ebenfalls nicht möglich ist, kommt bei Rhesus-negativen Frauen im gebärfähigen Alter der Prophylaxe der Rhesus-Sensibilisierung mit Immunglobulin Anti-D entscheidende Bedeutung zu.

Ohne Behandlung würden bis zu 17% der Primigravidae mit Rhesuskonstellation (Mutter Rhesus-negativ, Kind Rhesus-positiv) im Verlaufe ihrer Schwangerschaft oder während der Geburt gegen das Rhesus-Antigen sensibilisiert.

Anti-D Präparate werden seit über 30 Jahren erfolgreich eingesetzt zur Verhinderung der Rhesus-Sensibilisierung von Rhesus-negativen bzw. D^{u}-positiven Frauen gegen den Rhesusfaktor D bzw. D^{u} und damit der Anti-D-bedingten Neugeborenenkrankheiten Rhesus-Erythroblastose in all ihren Formen.

Das Risiko einer Rhesus-Sensibilisierung nimmt mit der Grösse der Einschwemmung foetaler Rhesus-positiver Erythrozyten zu. Die von der WHO empfohlene Behandlungsdosis von 200 µg Anti-D IgG als postpartale Prophylaxe reicht aus, um bis zu 10 ml foetale Erythrozyten (entsprechend ca. 20 ml foetalem Blut) zu neutralisieren und verhütet somit, selbst bei grösseren Einschwemmungen, in ca. 90% der Fälle eine Rhesus-Sensibilisierung; damit ist nur noch in 1-2% aller Primigravidae mit Rhesuskonstellation mit einer Sensibilisierung gegen das Rhesus-Antigen zu rechnen. Durch zusätzliche routinemässige Rhesusprophylaxe während der Schwangerschaft (antepartale Prophylaxe) kann das Sensibilisierungsrestrisiko noch einmal auf 0.1-0.2% aller Primigravidae mit Rhesuskonstellation vermindert werden.

Zusätzlich wird Anti-D auch verwendet nach Fehltransfusionen von Rhesus-positivem Blut auf Rhesus-negative Empfängerinnen und Empfänger; dabei ist die Dosierung dem Ausmass der Einschwemmung Rhesus-positiver Erythrozyten anzupassen.

Seit einigen Jahren wird die Anwendung von Anti-D Immunglobulin auch zur Behandlung der Idiopathischen thrombozytopenischen Purpura (ITP) diskutiert.

Die Pharmakokinetik von intramuskulär und intravenös injiziertem IgG im Organismus zeigt deutlich, dass die intravenöse Applikation unbedingt zu bevorzugen ist. Bei intramuskulärer Injektion muss infolge der langsamen Resorption aus dem Muskeldepot und lokaler Proteolyse mit einer zeitlichen Verzögerung der maximalen Aktivität und mit einer Wirkungseinbusse gerechnet werden. Die maximale IgG-Plasmakonzentration wird bei gesunden Probanden erst 4, bei bettlägerigen erst 6 Tage nach Injektion erreicht; zudem beträgt die maximale IgG-Plasmakonzentration bei gesunden Probanden nur rund 30%, bei bettlägerigen nur 20% der injizierten Dosis.

Demgegenüber bringt die intravenöse Anwendung wesentliche Vorteile. Nur bei dieser Anwendungsform kommt unabhängig von der körperlichen Aktivität die gesamte verabreichte Dosis sofort zur Wirkung; der IgG-Plasmaspiegel sinkt im Verlaufe von 5 Tagen, praktisch unabhängig von der körperlichen Aktivität, auf rund 35-40% der verabreichten Dosis ab, und erreicht damit erst am fünften Tage Werte, die mit der maximal erreichten Plasmakonzentration nach intramuskulärer Injektion vergleichbar sind. (Morell, A. et al. Vox Sang. *38*, 272, 1980).

Für die Herstellung von Immunglobulinpräparaten im Produktionsmassstab werden heute verschiedene Fraktionierverfahren angewendet:

Alkoholfällungen in der Kälte, zum Beispiel nach Cohn, oder modifizierte Verfahren nach Cohn sind vor allem geeignet für die Verarbeitung von Plasmamengen über 500 l pro Woche. Durch spezielle Behandlung, zum Beispiel mit Pepsin bei pH 4, können derart isolierte Immunglobuline intravenös verträglich gemacht werden. Andere Präzipitationsverfahren beruhen auf der spezifischen Ausfällung von Immunglobulinen durch Ammoniumsulfat, Natriumsulfat, Polyethylenglykol, Caprylsäure oder Rivanol (Übersicht s. Curling, J.M. in: Separation of plasma proteins, J.M. Curling ed., 1983, Pharmacia, Uppsala, Schweden).

Ein erstes Verfahren zur Isolierung von Immunglobulinen durch Batchadsorption an lonenaustauschern wurde bereits 1964 beschrieben (Baumstark, J.S. et al. Arch. Biochem. Biophys., *108*, 514, 1964). In den folgenden Jahren wurden auch eine Anzahl von Säulenverfahren entwickelt (CH Patent 572745 ; US Patent 3,869,436) und unter anderem auch für die Fraktionierung von Anti-D Immunglobulin angewendet (Friesen, A.D. et al. J. Appl. Biochem. *3*, 164,1981).

Bei all den bekannten Verfahren wurde bei hoher Reinheit eine hohe Ausbeute an IgG, aber keine spezifische Anreicherung bestimmter spezifischer IgG-Moleküle, wie zum Beispiel Anti-D, erreicht.

Die meisten Anti-D Hyperimmunglobulinpräparate sind nur für intramuskuläre Anwendung geeignet. Weltweit sind nur wenige intravenös verträgliche Präparate auf dem Markt. Ein solches Präparat ist das Immunglobulin Anti-D SRK der Anmelderin, das nach der Fraktioniermethode von Kistler und Nitschmann (Kistler, P. und Nitschmann, H. Vox Sang, 7, 414,1962) hergestellt und durch eine milde Pepsinbehandlung bei pH 4 intravenös verträglich gemacht wird. Da mit derartigen Fraktionierverfahren die Ausbeute der spezifischen Anti-D Antikörper gering ist, und gleichzeitig Anti-D Hyperimmunplasma rar ist, bestand ein Bedarf nach einem neuen Verfahren, welches, im Gegensatz zu den beschriebenen Methoden, auch mit niedrigtitrigen Anti-D Plasmapools, bei hoher Ausbeute ein reines IgG-Präparat mit hoher spezifischer Anti-D Aktivität ergibt.

In *Friesen, Journal of Applied Biochemistry, Vol. 3, 1981, p. 164* - *175,* wird ein Verfahren zur Reinigung von Anti-D (Anti-D-Rhesus) Immunglobulin G beschrieben. Menschliches Plasma wurde auf eine DEAE-Sephadex Säule gebracht, die zuerst mit einem 25 mM Phosphatpuffer, pH 7,5 äquilibriert worden war. Die ungebundenen Immunoglobuline wurden mit dem gleichen Puffer eluiert. Es wird erwähnt, dass die Immunoglobulinlösung, die mit Glycin und NaCI stabilisiert wurde, rein war und keine IgA oder IgM enhielt. Diese Immunoglobulinlösung ist für die intravenöse Anwendung geeignet.

Ein Ziel der vorliegenden Erfindung ist somit die Bereitstellung eines Verfahrens zur Herstellung eines reinen Anti-D Konzentrates, in welchem die Anti-D Immunglobuline der Klasse IgG spezifisch angereichert werden. Es wurde gefunden, dass dies auf überraschend einfache Art, bei gleichzeitiger Elimination von ca. 90% der unspezifischen IgG und nahezu aller unerwünschten anderen Plasmakomponenten geschehen kann, indem Blutplasma oder daraus gewonnene Immunglobulin enthaltende Fraktionen von speziell ausgewählten und spezifisch immunisierten Blutspenderinnen und Blutspendern, mit einem Titer von vorzugsweise mehr als 30 µg/ml Anti-D IgG, unter den erfindungsgemässen Bedingungen einer Kationenaustauschchromatographie unterzogen wird. Das in höherer Ausbeute erhaltene Anti-D Konzentrat besitzt zudem eine erhöhte spezifische Aktivität (Gramm Anti-D IgG pro Gramm Gesamt-IgG) und kann durch weitere Behandlungen mit Ionenaustauschern oder Aluminiumhydroxidgel, einzeln oder in geeigneter Kombination, weiter gereinigt werden.

Ein weiteres Ziel dieser Erfindung ist eine Anti-D Präparation, die Immunglobulin A nur in Spuren enthält, intravenös verträglich ist und mit Zusatz von geeigneten Stabilisatoren, wahlweise lyophilisiert oder vorzugsweise in Lösung, ohne Aktivitätseinbusse während mehrerer Monate gelagert werden kann. Es wurde gefunden, dass das durch das erfindungsgemäss vorgeschlagene Verfahren erhaltene, reine Anti-D Konzentrat diese Anforderungen erfüllt, d.h. dass es u.a. eine bisher nicht erreichte spezifische Aktivität aufweist, intravenös verträglich ist und einen extrem geringen Gehalt an IgA aufweist.

Gegenstand der vorliegenden Erfindung ist demzufolge das im Patentanspruch 1 definierte Verfahren.

Im erfindungsgemässen Verfahren wird Immunglobulin Anti-D aus humanem Plasma hergestellt. Ausgangsmaterial ist das Plasma von Rhesus-negativen gegen den Rhesusfaktor D sensibilisierten Spenderinnen und Spendern. Vorzugsweise wird die durch Plasmapherese gewonnene Einzelspende eingefroren und vor der Fraktionierung sorgfältig bei 0-4°C aufgetaut und gepoolt. Der Plasmapool sollte einen Gehalt von mehr als 10 µg Anti-D IgG pro ml, vorzugsweise einen Gehalt von mehr als 30 µg Anti-D IgG pro ml aufweisen. Die für die Kationenaustauschchromatographie verwendete Plasmafraktion kann nach irgend einem bekannten Verfahren, beispielsweise durch Abtrennung der Kryoglobuline oder durch fraktionierte Fällung mittels Alkohol (Cohn, E.J. et al. J. Am. Chem. Soc. *68,* 459, 1949; Kistler, P. und Nitschmann, H. Vox Sang. *7*, 414, 1962), durch andere Präzipitationsverfahren, wie Ausfällung der Immunglobuline durch Ammoniumsulfat, Natriumsulfat, Polyethylenglykol, Caprylsäure oder Rivanol oder einem chromatographischen Verfahren oder einer Kombination solcher Verfahren vorgereinigt werden. Vorzugsweise wird vor der erfindungsgemässen Kationenaustauschchromatographie das kryoglobulinfreie Plasma oder die, im Äquilibrierpuffer aufgelöste, Immunglobulin enthaltende Plasmafraktion filtriert und zur Inaktivierung von behüllten Viren mit einem biokompatiblen organischen Lösungsmittel, wie zum Beispiel Tri-n-butylphosphat und einem Detergens, wie zum Beispiel O-[4-(1,1,3,3-Tetramethylbutyl)-phenyl]-deca(oxyethylen) (Triton® X-100) nach Horowitz (Thrombosis and Haemostasis *65*, 1163, 1991) behandelt. Das Plasma-Lösungsmittel-Detergens-Gemisch wird anschliessend bei 37°C inkubiert. Dabei erfolgt eine Phasentrennung. Der klare Unterstand wird abgeschieden, verdünnt, filtriert und auf die Äquilibrierbedingungen der Kationenaustauschchromatographie eingestellt, wobei die Temperatur für die weiteren Schritte in der Regel oberhalb von 10°C liegt und vorzugsweise 20-25°C beträgt. In einem ersten Hauptreinigungsschritt wird Anti-D IgG aus dem vorbehandelten Produkt an ein schwach saures lonenaustauschergel mit vorzugsweise Carboxymethyl (CM) als funktionelle Gruppe gebunden. Fremdproteine werden dabei grösstenteils weggewaschen; ebenfalls werden die zur Vireninaktivierung verwendeten Lösungsmittel und Detergenzien hier entfernt. Das durch diesen Verfahrensschritt spezifisch angereicherte Anti-D IgG wird, durch Erhöhung der Leitfähigkeit auf vorzugsweise über 10 mS/cm, vom lonenaustauschergel eluiert und anschliessend durch Behandlung mit einem zweiten, schwach basischen lonenaustauschergel, mit vorzugsweise Diethylaminoethyl (DEAE) als funktioneller Gruppe, weiter gereinigt, wobei unter den gewählten Bedingungen Anti-D IgG nicht an den Ionenaustauscher bindet. Die gereinigte Anti-D IgG-Fraktion wird zum Aufkonzentrieren vorzugsweise ein zweitesmal an ein CM-lonenaustauschergel gebunden und unter geeigneten Bedingungen möglichst konzentriert eluiert und zum Endprodukt konfektioniert. Es ist für die Erreichung der erfindungsgemässen Bedingungen nicht von Bedeutung, ob Anti-D IgG durch Erhöhung der lonenstärke, oder Verschiebung des pH oder durch einen in der Zusammensetzung veränderten Puffer eluiert wird. Um weitere unerwünschte Komponenten, wie zum Beispiel Proteasen, weiter abzureichern, kann die Immunglobulinlösung auf irgendeiner Stufe des Verfahrens, zusätzlich mit einem Adsorbens, wie zum Beispiel Aluminiumhydroxidgel, behandelt werden. Die Lösungsmittel-Detergens-Behandlung kann wahlweise durch einen anderen bekannten Vireninaktivierungsschritt, beispielsweise eine Hitzebehandlung, wie zum Beispiel eine Pasteurisierung, ersetzt oder ergänzt werden.

Ein Anti-D IgG, das erfindungsgemäss hergestellt wird, ist flüssig stabil. Es ist weiter intravenös verträglich, besitzt einen IgA-Gehalt von weniger als 5 µg/ml und hat einen IgG-Gehalt von 1-10 mg/ml. Der pH-Wert liegt bei ca. 5.2. Vorzugsweise enthält das Präparat bis zu 25 mM Phosphat, bis zu 200 mM NaCI und bis zu 100 mg/ml humanes Albumin und wahlweise 250-300 mM einer Aminosäure, wie zum Beispiel Glycin oder bis zu 100 mg/ml eines Disaccharids, wie zum Beispiel Saccharose oder 50 mg/ml eines Monosaccharids, wie zum Beispiel Mannit.

Ein Anti-D IgG, das erfindungsgemäss hergestellt wird, kann alternativ unter Zusatz eines Disaccharids, wie beispielsweise 100 mg/ml Saccharose, in einer Dosis von 200 µg Anti-D IgG bei einem pH von ca. 6.6 lyophilisiert werden.

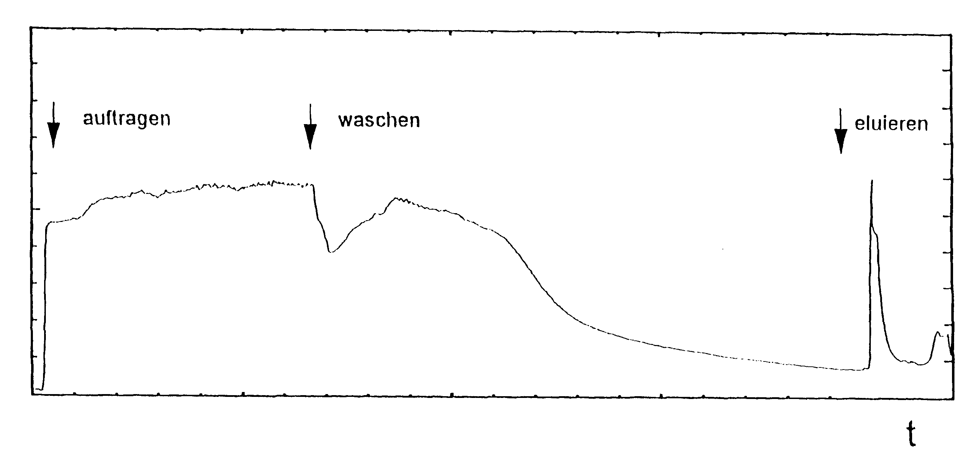

Die beiliegende Zeichnung dient zur Erläuterung der vorliegenden Erfindung, ohne dass dadurch eine Einschränkung beabsichtigt ist. Sie zeigt das typische Chromatogramm der ersten Kationenaustauschchromatographie gemäss Beispiel 1.

Das erfindungsgemässe Verfahren wird anhand der folgenden Beispiele näher erläutert.

### Beispiel 1

Ausgangsmaterial ist das Plasma von Rhesus-negativen Frauen oder von Rhesus-negativen Männern, die sich mit sorgfältig ausgewählten Erythrozyten gegen den Rhesusfaktor D sensibilisieren liessen. Jede Blutspende wird auf die Abwesenheit von HBs-Antigen, HIV- und Hepatitis C Antikörpern und erhöhter ALAT-Aktivität getestet. Das durch Plasmapherese gewonnene Plasma wird einzeln eingefroren und vor der Fraktionierung sorgfältig bei 0-4°C aufgetaut und gepoolt. Der eisfreie Plasmapool wird in einer Durchlaufzentrifuge (Cepa, Lahr, Deutschland) bei 2-4°C zentrifugiert(1 l/min; 12000 U/min). Der Überstand wird durch ein 1.2 µm Filter (Opticap®, Millipore, Bedford MA, USA) filtriert. Anschliessend erfolgt die Vireninaktivierung nach Horowitz et al. (Thrombosis and Haemostasis *65*,1163,1991) durch eine Lösungsmittel-Detergens-Behandlung mit 1% Triton® X-100 (Rohm und Haas, Frankfurt, Deutschland) und 1% tri-n-Butylphosphat (Merck, Darmstadt, Deutschland) während 4-4.5 Stunden bei 30°C. Die vireninaktivierte Lösung wird anschliessend während 10-18 Stunden bei 37°C stehen gelassen. Der klare Unterstand wird abgetrennt und durch ein 0.2µm Filter (Sealkleen NFP 7002, Pall, Dreieich, Deutschland) filtriert. 25l Lösungsmittel-Detergens behandeltes, filtriertes Plasma werden mit einem 10 mM Natriumphosphatpuffer auf die Leitfähigkeit des Äquilibrierpuffers (50 mM Natriumphosphatpuffer, pH 5.5, 3.2 mS/cm) verdünnt, das pH auf 5.5 eingestellt, durch ein 1.2 µm Filter (Opticap®, Millipore, Bedford MA, USA) filtriert und in einer Säule, mit einer Grundfläche von 314 cm² und einer Betthöhe von 16 cm, mit einer Flussrate von 150 cm/h an MacroPrep® 50 CM (BioRad, Hercules CA, USA) gebunden, wobei das Gel zuvor mit 50 mM Natriumphosphatpuffer, pH 5.5 äquilibriert worden ist. Die Temperatur wird auf 20-25°C gehalten. Die Säule wird anschliessend mit 40 Säulenvolumen 25 mM Natriumphosphatpuffer, pH 7.0 gewaschen und die Anti-D enthaltende IgG-Fraktion mit 25 mM Natriumphosphatpuffer + 0.2 M Natriumchlorid, pH 7.5 eluiert. Das typische Chromatogramm, wie es durch die vorliegende Behandlung erhalten wird ist in der einzigen Figur dargestellt. Im Diagramm ist die optische Dichte (OD) bei 280 nm gegen die Zeit (t) aufgetragen. Daraus ist ersichtlich, dass während des Auftragens und des Waschens der grösste Teil der unerwünschten Produkte abgetrennt wird, während bei der Elution ein schmaler Hauptpeak in Erscheinung tritt, welcher das erwünschte Anti-D IgG enthält. Das Eluat wird mit Wasser auf eine Leitfähigkeit von 3.3 mS/cm verdünnt (1 Volumen Eluat + ca. 5 Volumen Wasser), das pH mit 0.2 M NaOH auf 7.5 eingestellt und im Batch-Verfahren während 2.5 Stunden mit 100 g DEAE-Sephadex® A50 trocken (Pharmacia, Uppsala, Schweden), adsorbiert. Das DEAE-Gel wird zuvor mit einem 25 mM Natriumphosphatpuffer, pH 7.5 äquilibriert. Die ungebundene Anti-D IgG Fraktion wird durch ein Polypropylengewebe abfiltriert und das pH auf 5.5 eingestellt. Das DEAE-Filtrat wird zum Aufkonzentrieren ein zweitesmal an MacroPrep® 50 CM (BioRad. Hercules CA, USA) in einer Säule, mit einer Grundfläche von 78.5 cm² und einer Betthöhe von 10 cm, mit einer Flussrate von 60 cm/h gebunden, wobei das Gel zuvor mit 50 mM Natriumphosphatpuffer, pH 5.5 äquilibriert wird. Die Anti-D enthaltende IgG-Fraktion wird mit 25 mM Natriumphosphatpuffer + 0.2 M Natriumchlorid, pH 5.5 eluiert. Die CM-Gele werden mit 1 N NaOH regeneriert und pyrogenfrei gewaschen und können mindestens zwanzigmal wiederverwendet werden; das DEAE-Gel wird nach einmaligem Gebrauch verworfen.

### Beispiel 2

Ausgangsmaterial ist das Plasma von Rhesus-negativen Frauen oder von Rhesus-negativen Männern, die sich mit sorgfältig ausgewählten Erythrozyten gegen den Rhesusfaktor D sensibilisieren liessen. Jede Blutspende wird auf die Abwesenheit von HBs-Antigen, HIV- und Hepatitis C Antikörpern und erhöhter ALAT-Aktivität getestet. Das durch Plasmapherese gewonnene Plasma wird einzeln eingefroren und vor der Fraktionierung sorgfältig bei 0-4°C aufgetaut und gepoolt. Der eisfreie Plasmapool wird in einer Durchlaufzentrifuge (Cepa, Lahr, Deutschland) bei 2-4°C zentrifugiert (1 l/min; 12000 U/min). Der Überstand wird durch ein 1.2 µm Filter (Opticap®, Millipore, Bedford MA, USA) filtriert. Anschliessend erfolgt die Vireninaktivierung nach Horowitz et al. (Thrombosis and Haemostasis *65*, 1163, 1991) durch eine Lösungsmittel-Detergens-Behandlung mit 1 % Triton® X-100 (Rohm und Haas, Frankfurt, Deutschland) und 1% tri-n-Butylphosphat (Merck, Darmstadt, Deutschland) während 4-4.5 Stunden bei 30°C. Die vireninaktivierte Lösung wird anschliessend während 10-18 Stunden bei 37°C stehen gelassen. Der klare Unterstand wird abgetrennt und durch ein 0.2 µm Filter (Sealkleen NFP 7002, Pall, Dreieich, Deutschland) filtriert. 25 l Lösungsmittel-Detergens behandeltes, filtriertes Plasma werden mit einem 10 mM Natriumphosphatpuffer auf die Leitfähigkeit des Äquilibrierpuffers (50 mM Natriumphosphatpuffer, pH 5.5, 3.2 mS/cm) verdünnt, das pH auf 5.5 eingestellt, durch ein 1.2 µm Filter (Opticap®, Millipore, Bedford MA, USA) filtriert und in einer Säule, mit einer Grundfläche von 314 cm² und einer Betthöhe von 16 cm, mit einer Flussrate von 150 cm/h an MacroPrep® 50 CM (BioRad, Hercules CA, USA) gebunden, wobei das Gel zuvor mit 50 mM Natriumphosphatpuffer, pH 5.5 äquilibriert worden ist. Die Temperatur wird auf 20-25°C gehalten. Die Säule wird anschliessend mit 40 Säulenvolumen 25 mM Natriumphosphatpuffer, pH 7.0 gewaschen und die Anti-D enthaltende IgG-Fraktion mit 25 mM Natriumphosphatpuffer + 0.2 M Natriumchlorid, pH 7.5 eluiert. Das Eluat wird bei pH 6.6 unter Zusatz von 100 mg/ml Saccharose in einer Dosis von 200 µg Anti-D IgG durch ein 0.2 µm Filter (Millidisk® MCGL-10, Millipore, Bedford MA, USA) filtriert und anschliessend lyophilisiert.

### Beispiel 3

5 ml Lösungsmittel-Detergens behandeltes Plasma wird auf die Leitfähigkeit des Äquilibrierpuffers (3.3 mS/cm) verdünnt und in einer Säule, mit einer Grundfläche von 2 cm² und einer Betthöhe von 5 cm, mit einer Flussrate von 30 cm/h über DEAE-Sephadex® A50 (Pharmacia, Uppsala, Schweden) chromatogratiert. Das Gel wird zuvor mit einem 25 mM Natriumphosphatpuffer, pH 7.5 äquilibriert. Die Temperatur wird auf ca. 20-25°C gehalten. Die ungebundene IgG Fraktion wird zur weiteren Reinigung mit 0.2 M HCI auf pH 5.5 und eine Leitfähigkeit von 3.2 mS/cm eingestellt und in einer Säule, mit einer Grundfläche von 0.8 cm² und einer Betthöhe von 6.5 cm, mit einer Flussrate von 230 cm/h an MacroPrep® 50 CM (BioRad, Hercules CA, USA) gebunden, wobei das Gel zuvor mit 50 mM Natriumphosphatpuffer, pH 5.5 äquilibriert worden ist. Die Anti-D enthaltende IgG-Fraktion wird mit 25 mM Natriumphosphatpuffer + 0.2 M Natriumchlorid, pH 5.5 eluiert und zum Endprodukt konfektioniert. Das DEAE-Sephadex® A50-Gel wird nach einmaligem Gebrauch verworfen; das MacroPrep® 50 CM-Gel wird mit 1 N NaOH regeneriert und pyrogenfrei gewaschen und kann mindestens zwanzigmal wiederverwendet werden.

### Beispiel 4

Die Verfahren gemäss Beispiel 1 bis 3 werden wiederholt, wobei als Ausgangsmaterial anstelle von Plasma, eine Immunglobulin enthaltende Plasmafraktion verwendet wird, die aus einer entsprechenden Menge Ausgangsplasma hergestellt wird, indem Fraktion I+II+III oder Fraktion II+III nach Cohn (Cohn, E.J. et al. J. Am. Chem. Soc. *68*, 459, 1949) oder Niederschlag A oder Niederschlag Gammaglobulin nach Kistler und Nitschmann (Kistler, P. und Nitschmann, H. Vox Sang, *7*, 414, 1962) gewonnen und im Äquilibrierpuffer ad 30 g/l Protein gelöst werden, wobei analoge Ergebnisse erhalten werden.

### Beispiel 5

Die Verfahren gemäss Beispiel 1 bis 4 werden wiederholt, wobei die Anti-D enthaltende IgG-Fraktion alternativ anstatt mit 25 mM Natriumphosphatpuffer + 0.2 M Natriumchlorid, pH 7.5, mit 0.1 M Glycin + 0.5 M Natriumchlorid, pH 9 oder generell durch pH-Verschiebung und/oder Veränderung der lonenstärke und/oder Veränderungen der Pufferzusammensetzung vom MacroPrep® 50 CM Gel (BioRad, Hercules CA, USA) eluiert werden, wobei analoge Ergebnisse erhalten werden.

### Beispiel 6

Die Verfahren gemäss Beispiel 1 und 2 werden wiederholt, wobei das MacroPrep® 50 CM Gel (BioRad, Hercules CA, USA) alternativ anstatt mit 25 mM Natriumphosphatpuffer pH 7.0, mit 10 mM Glycin, pH 9 gewaschen wird, wobei analoge Ergebnisse erhalten werden.

### Beispiel 7

Das nach Beispiel 1 erhaltene konzentrierte Anti-D Eluat wird auf folgende Bedingungen eingestellt:

| | flüssiges Präparat I | flüssiges Präparat II | flüssiges Präparat III | flüssiges Präparat IV |
|---|---|---|---|---|
| Anti-D | 100 (µg/ml | 100 µg/ml | 100 µg/ml | 100 µg/ml |
| Glycin | 20.6 mg/ml | 20.6 mg/ml | 20.6 mg/ml | 20.6 mg/ml |
| Albumin | --- | 10 mg/ml | 50 mg/ml | 100 mg/ml |
| pH | 5.20 | 5.20 | 5.20 | 5.20 |
| Dosis | 2 ml | 2 ml | 2 ml | 2 ml |
| Dosis | 2 ml | 2 ml | 2 ml | 2 ml |

| | flüssiges Präparat V | flüssiges Präparat VI | flüssiges Präparat VII | flüssiges Präparat VIII |
|---|---|---|---|---|
| Anti-D | 100 µg/ml | 100 µg/ml | 100 µg/ml | 100 µg/ml |
| D-Mannit | 50 mg/ml | 50 mg/ml | 50 mg/ml | 50 mg/ml |
| Albumin | --- | 10 mg/ml | 50 mg/ml | 100 mg/ml |
| pH | 5.20 | 5.20 | 5.20 | 5.20 |
| Dosis | 2 ml | 2 ml | 2 ml | 2 ml |

und nach Filtration durch ein 0.2 µm Filter (Millipak®-20, Millipore, Bedford MA, USA) in Hypac Fertigspritzen aus Glas (Vetter, Ravensburg, Deutschland) steril abgefüllt.

### Beispiel 8

Das DEAE-Filtrat nach Beispiel 1 wird bei einem pH von ca. 5.5 mit 0.2g Aluminiumhydroxidgel pro g Protein während 30 Minuten bei 20-25°C behandelt und anschliessend nach Beispiel 1 weiter aufbereitet.

### Beispiel 9

Die Verfahren gemäss Beispiel 1 bis 6 werden wiederholt, wobei alternativ anstelle von MacroPrep® 50 CM eines der folgenden Gele mit der gleichen funktionellen Gruppe verwendet wird: CM-Spherodex® (Sepracor IBF, Villeneuve la Garenne, Frankreich), CM-Trisacryl® (Sepracor IBF, Villeneuve la Garenne, Frankreich), CM-Sepharose® FF (Pharmacia, Uppsala, Schweden) oder Fractogel® TSK CM-650 (Merck, Darmstadt, Deutschland), wobei analoge Ergebnisse erhalten werden.

### Beispiel 10

Die Verfahren gemäss Beispiel 1, sowie 3 bis 6 werden wiederholt, wobei alternativ anstelle von DEAE-Sephadex® A 50 MacroPrep® DEAE (BioRad, Hercules CA, USA) verwendet wird, wobei analoge Ergebnisse erhalten werden.

Die chromatographischen Verfahrensschritte in den obigen Beispielen 1 bis 6 können wahlweise als Batch-, Säulen- oder Membranchromatographien durchgeführt werden, wobei analoge Ergebnisse erhalten werden.

## Patentansprüche

1. Verfahren zur Herstellung eines Anti-D Immunglobulin G-Präparates mit einer spezifischen Aktivität von mehr als 0,5 % Anti-D IgG pro Gramm Gesamt-IgG,aus humanem Plasma, **dadurch gekennzeichnet, dass** Plasma oder eine Anti-D IgG enthaltende Plasmafraktion aus Rhesusnegativem Blut von Rhesusfaktor D sensibilisierten Spenderinnen und Spendern
A) bei einem pH-Wert im Bereich von pH 3,5 bis 6,5 einer lonenaustauscherchromatographie unterworfen wird, mit einem Adsorbens, das Carboxymethyl-Gruppen als funktionelle Gruppen aufweist, wobei das Anti-D IgG am Adsorbens gebunden wird,
B) das Adsorbens mit dem gebundenen Anti-D IgG zuerst mit einer Waschlösung gespult wird, und anschließend das Anti-D IgG eluiert wird, und weiter
C) das eluierte Anti-D IgG mit einem basischen Adsorbens mit lonenaustausch-Eigenschaften behandelt wird, um unerwünschte Komponenten zu binden, und zuletzt das Anti-D IgG konzentriert wird.

2. Verfahren nach Anspruch 1 **dadurch gekennzeichnet, dass** in Stufe C) das Anti-D IgG konzentriert wird, indem die Schritte A) und B) mindestens einmal wiederholt werden.

3. Verfahren nach Anspruch 1 oder 2 **dadurch gekennzeichnet, dass** Plasma als Ausgangsmaterial verwendet wird, welches mit einem basischen Adsorbens mit Ionenaustausch-Eigenschaften vorbehandelt wurde, um unerwünschte Komponenten zu binden.

4. Verfahren nach Anspruch 1 oder 2 **dadurch gekennzeichnet, dass** der Proteasegehalt im Plasma oder in der Plasmafraktion durch Inkubation mit einem Adsorbens, wie Aluminiumhydroxidgel, vermindert wird.

5. Verfahren nach einem der Ansprüche 1 bis 4, **dadurch gekennzeichnet, dass** alle lonenaustauschchromatographieschritte und gegebenenfalls weitere Adsorptionsschritte wahlweise als Batch-, Säulen- oder Membranchromatographie durchgeführt werden.

6. Verfahren nach einem der Ansprüche 1 bis 5 **dadurch gekennzeichnet, dass** das basische Adsorbens in Schritt C) Diethylaminoethyl-Gruppen als funktionelle Gruppen aufweist.

7. Verfahren nach einem der Ansprüche 1 bis 6 **dadurch gekennzeichnet, dass** die Elution im Schritt B) unter pH-Verschiebung und/oder Veränderung der Pufferzusammensetzung, beispielsweise durch Veränderung ihrer lonenstärke bzw. Leitfähigkeit durchgeführt wird.

8. Verfahren nach einem der Ansprüche 1 bis 7 **dadurch gekennzeichnet, dass** es mindestens einen Vireninaktivierungsschritt umfasst.

9. Verfahren nach Anspruch 8 **dadurch gekennzeichnet, dass** der Vireninaktivierungsschritt eine Behandlung des Plasmas oder der Anti-D IgG enthaltenden Fraktion mit einem Detergens und Tri-n-butylphosphat umfasst, wobei die Phasen des Lösungsmittel und Detergens enthaltenden Gemischs getrennt werden und der klare Unterstand verwendet wird.

10. Verfahren nach Anspruch 8 **dadurch gekennzeichnet, dass** der Vireninaktivierungsschritt eine Hitzebehandlung ist.

11. Verfahren nach einem der Ansprüche 1 bis 10 **dadurch gekennzeichnet, dass** das Ausgangsplasma oder die Ausgangsplasmafraktion vor dem lonenaustauschchromatographieschritt in Stufe A) mindestens einem der nachstehend erwähnten Schritte unterworfen wird:
a) Einfrieren des Plasmas, wobei nach dem Auftauen vor seiner Weiterverwendung das Kryopräzipitat durch Filtration und/oder Zentrifugation abgetrennt wird
b) Behandlung mit einem Lösungsmittel-Detergens-Gemisch, Inkubation bei ca. 37°C und Phasentrennung

## Claims

1. Method of producing an anti-D immunoglobulin G-preparation with a specific activity of more than 0.5% anti-D IgG per gram total IgG, from human plasma, **characterised in that** plasma or an anti-D IgG containing plasma fraction from rhesus negative blood of rhesus factor D sensitised donors:
A) with a pH value in the range of pH 3.5 to 6.5 is subjected to an ion exchange chromatography, with an adsorbent which has carboxymethyl groups as functional groups, the anti-D IgG being bound to the adsorbent,
B) the adsorbent with the bound anti-D IgG is first rinsed with a wash solution, and the anti-D IgG is subsequently eluted, and further
C) the eluted anti-D IgG is treated with an alkaline adsorbent with ion-exchange characteristics in order to bind undesired components, and finally the anti-D IgG is concentrated.

2. Method according to claim 1, **characterised in that** the anti-D IgG is concentrated in stage C), **in that** steps A) and B) are repeated at least once.

3. Method according to claim 1 or 2, **characterised in that** used as the starting material is plasma which has been pre-treated with a basic adsorbent with ion-exchange characteristics in order to bind undesired components.

4. Method according to claim 1 or 2 **characterised in that** the protease content in the plasma or in the plasma fraction is reduced through incubation with an adsorbent such as aluminium hydroxide gel.

5. Method according to one of the claims 1 to 4, **characterised in that** all ion exchange chromatography steps, and if necessary further adsorption steps, are carried out alternatively as batch chromatography, column chromatography or membrane chromatography.

6. Method according to one of the claims 1 to 5, **characterised in that** the alkaline adsorbent in step C) has diethylaminoethyl groups as functional groups.

7. Method according to one of the claims 1 to 6, **characterised in that** the elution in step B) is carried out with pH shift and/or change of the buffer composition, for example by changing its ionic concentration or conductivity, respectively.

8. Method according to one of the claims 1 to 7, **characterised in that** it comprises at least one step of virus inactivation.

9. Method according to claim 8, **characterised in that** the step of virus inactivation comprises treatment of the plasma or of the fraction containing anti-D IgG with a detergent and tri(n-butyl) phosphate, the phases of the mixture containing solvent and detergent being separated and the clear lower phase being used.

10. Method according to claim 8, **characterised in that** the step of virus inactivation is a heat treatment.

11. Method according to one of the claims 1 to 10, **characterised in that** the starting plasma or the starting plasma fraction prior to the ion exchange chromatography step in stage A) is subjected to at least one of the steps mentioned in the following:
a) freezing the plasma, the cryoprecipitate being separated by filtration and/or centrifugation after thawing and before its further use
b) treatment with a solvent-detergent-mixture, incubation at approximately 37° C and phase separation.

## Revendications

1. Procédé d'obtention d'une préparation d'immunoglobuline G anti-D, à partir de plasma humain, ayant une activité spécifique de plus de 0,5 % d'IgG anti-D par gramme du total d'lgG, **caractérisé en ce que** du plasma ou une fraction de plasma contenant d'IgG anti-D provenant de sang rhésus négatif de donneuses et donneurs sensibilisés au facteur rhésus D
A) est soumis à une chromatographie par échanges d'ions à une valeur de pH de l'ordre de pH 3,5 à 6,5 avec un adsorbant présentant des groupes carboxyméthyle comme groupes fonctionnels, l'IgG anti-D étant liée à l'absorbant,
B) l'adsorbant avec l'IgG anti-D liée est d'abord rincé avec une solution de lavage ensuite de quoi l'IgG anti-D est éluée et puis
C) l'IgG anti-D éluée est traitée avec un adsorbant basique ayant des propriétés d'échanges d'ions afin de lier les composants non désirés et l'IgG anti-D est finalement concentrée.

2. Procédé selon la revendication 1, **caractérisé en ce que** l'IgG anti-D est concentrée à l'étape C) alors que les étapes A) et B) sont répétées au moins une fois.

3. Procédé selon la revendication 1 ou la revendication 2, **caractérisé en ce que** comme matériau de départ on utilise du plasma qui a été préalablement traité avec un adsorbant basique ayant des propriétés d'échanges d'ions pour lier les composants non désirés.

4. Procédé selon la revendication 1 ou la revendication 2, **caractérisé en ce qu**on réduit le contenu de protease dans le plasma ou dans la fraction de plasma par incubation avec un adsorbant, comme un gel d'hydroxyde d'aluminium.

5. Procédé selon lune des revendications 1 à 4, **caractérisé en ce que** tous les pas de chromatographie par échanges d'ions et, le cas échéant, les autres pas d'adsorption sont effectués facultativement comme chromatographie sur batch, colonne ou membrane.

6. Procédé selon lune des revendications 1 à 5, **caractérisé en ce que** l'adsorbant basique à l'étape C) présente des groupes diéthylaminoéthyle comme groupes fonctionnels.

7. Procédé selon lune des revendications 1 à 6, **caractérisé en ce qu'**on effectue lélution à létape B) en déplaçant le pH et/ou en changeant la composition du tampon, par exemple en modifiant sa concentration ionique, respectivement sa conductibilité.

8. Procédé selon l'une des revendications 1 à 7, **caractérisé en ce qu'**il comprend au moins un pas d'inactivation virale.

9. Procédé selon la revendication 8, **caractérisé en ce que** le pas d'inactivation virale comprend un traitement du plasma ou de la fraction de plasma contenant l'IgG anti-D avec un détergent et du phosphate de tri-n-butyle, les phases du mélange contenant du solvant et du détergent étant séparées et la phase inférieure claire étant utilisée.

10. Procédé selon la revendication 8, **caractérisé en ce que** le pas d'inactivation virale est un traitement à chaud.

11. Procédé selon lune des revendications 1 à 10, **caractérisé en ce que** le plasma de départ ou la fraction de plasma de départ, avant le pas de chromatographie par échanges d'ions à l'étape A) est soumis au moins à l'un des pas mentionnés ci-après:
a) congélation du plasma, le cryoprécipité étant séparé par filtration et/ou par centrifugation après décongélation et avant son emploi ultérieur
b) traitement avec un mélange de solvant et détergent, incubation à environ 37° et séparation des phases.
